# EUROPEAN PATENT SPECIFICATION

(11) **EP 2 702 989 B1**
(45) Date of publication and mention of the grant of the patent: **23.08.2017**
(21) Application number: 12777745.6
(22) Date of filing: 01.05.2012
(51) Int. Cl.: A61K 9/16, A61K 9/20, A61K 9/50, A61K 47/22, A61K 31/192, A61K 31/46, A61K 31/522, A61K 31/17, A61K 31/195, A61K 31/167, A61P 25/02, A61P 29/00, A61P 43/00

(54) **STABLE PHARMACEUTICAL COMPOSITION**
STABILE PHARMAZEUTISCHE ZUSAMMENSETZUNG
COMPOSITION PHARMACEUTIQUE STABLE

(30) Priority: 28.04.2011 JP 2011101363
(43) Date of publication of application: 05.03.2014
(73) Proprietor: Kowa Company, Ltd., Nagoya-shi, Aichi 460-8625 (JP)
(72) Inventor: KANO, Yuichiro, Fuji-shi Shizuoka 417-8650 (JP)
(74) Representative: Blodig, Wolfgang
(86) International application number: PCT/JP2012/061560
(87) International publication number: WO 2012/147986

(56) References cited:
- EP-A1- 2 659 889
- JP-A- 59 104 315
- JP-A- S59 501 459
- JP-A- 2000 026 313
- JP-A- 2000 344 665
- JP-A- 2003 277 258
- JP-A- 2004 189 653
- JP-A- 2007 284 423
- JP-A- 2008 115 167

## Description

### Field of the Invention

The present invention relates to a stable pharmaceutical composition and, more particularly, to a stable pharmaceutical composition containing ibuprofen and butylscopolamine. The present invention is defined in the appended claims. Subject matters which are not encompassed by the scope of the claims do not form part of the present invention.

### Background of the Invention

Ibuprofen is a non-steroidal anti-inflammatory drug and is known to serve as an anti-inflammatory, analgesic, and antipyretic pharmaceutical product effective for rheumatoid arthritis, arthralgia, arthritis, neuralgia, neuritis, back pain, cervicobrachial syndrome, utero-adnexitis, dysmenorrhea, acute upper respiratory inflammation, postoperative and posttraumatic pain, etc.

Also, ibuprofen is a compound which has been switched to an OTC drug, and is now widely used as an OTC drug (Non-Patent Documents 1 and 2).

However, ibuprofen, a type of NSAID, may cause adverse side effects including digestive tract damage. Thus, such side effects prevent an increase in dose of ibuprofen for obtaining higher antipyretic and analgesic effects, which is problematic.

In order to solve the above problem, there has been proposed administration of ibuprofen with scopolamine butylbromide, which is an analgesic and antispastic compound (Patent Document 1). Through the proposed technique, excellent analgesic effect can be obtained by use of ibuprofen at only a low dose.

Although a tablet preparation and a fine-granule preparation containing ibuprofen in combination with scopolamine butylbromide are known (Patent Document 1), it has not been clarified whether or not ibuprofen and scopolamine butylbromide show some interaction, or whether or not the tablet preparation and the fine-granule preparation are stable.

### Prior Art Documents

### Patent Documents

Patent Document 1: JP-A-2000-344665

### Non-Patent Documents

Non-Patent Document 1: OTC Handbook 2008-09, Product list, p. 2-9, edited by SIDIC
Non-Patent Document 2: OTC Handbook 2008-09, Product list, p. 16-39, edited by SIDIC

### Summary of the Invention

### Problems to be Solved by the Invention

The present inventors have studied to prepare a solid pharmaceutical composition containing ibuprofen and scopolamine butylbromide. Surprisingly, the inventors have found that, when stored as a mixture, some interaction between ibuprofen and scopolamine butylbromide is observed, and that the mixture undergoes discoloration, wetting, solidification, etc. due to the interaction, resulting in storage instability.

Thus, an object of the present invention is to provide a stable pharmaceutical composition containing ibuprofen and scopolamine butylbromide.

### Means for Solving the Problems

In order to solve the aforementioned object, the present inventors have investigated storage stability of a pharmaceutical composition containing ibuprofen and scopolamine butylbromide, and have found that the interaction between ibuprofen and scopolamine butylbromide can be reduced through co-presence of the two compounds with one or more species selected from the group consisting of a xanthine derivative, tranexamic acid or a salt thereof, acetaminophene, and an isovaleryl urea derivative. The present invention has been accomplished on the basis of this finding.

Accordingly, the present invention provides a pharmaceutical composition according to claims 1 to 6 comprising ibuprofen, scopolamine butylbromide, and one or more species selected from the group consisting of caffeine, tranexamic acid or a salt thereof, acetaminophene, and an isovaleryl urea derivative which is bromovaleryl urea or allylisopropylacetyl urea.

### Effects of the Invention

According to the present invention, one or more species selected from the group consisting of caffeine, tranexamic acid or a salt thereof, acetaminophene, and an isovaleryl urea derivative which is bromovaleryl urea or allylisopropylacetyl urea, are incorporated into a pharmaceutical composition containing ibuprofen and scopolamine butylbromide, whereby the interaction between the two compounds can be reduced. Thus, the present invention enables provision of a pharmaceutical composition containing ibuprofen and scopolamine butylbromide and showing excellent storage stability.

### Detailed Description of the Invention

The pharmaceutical composition of the present invention comprises ibuprofen, scopolamine butylbromide, and one or more species selected from the group consisting of caffeine, tranexamic acid or a salt thereof, acetaminophene, and an isovaleryl urea derivative which is bromovaleryl urea or allylisopropylacetyl urea.

The ingredients employed in the present invention are described hereinafter in detail.

Ibuprofen, employed in the pharmaceutical composition of the present invention, is a known compound, and may be produced through a known method or may be commercially available. In the present invention, ibuprofen listed in an official document or the like published in countries, regions, etc. is preferred. Examples of the official document include the Japanese Pharmacopoeia, the United States Pharmacopoeia, the British Pharmacopoeia, the European Pharmacopoeia, and the Pharmacopoeia of the People's Republic of China. Ibuprofen listed in the Japanese Pharmacopoeia assumes white crystalline powder.

The ibuprofen amount of the pharmaceutical composition of the present invention may be determined in accordance with the sex, age, symptoms, etc. of a patient in need of the composition and in appropriate consideration of the daily dose. The ibuprofen amount is preferably adjusted so as to attain an ibuprofen daily dose of 10 to 3,000 mg, more preferably 30 to 2,000 mg, still more preferably 100 to 600 mg. The daily dose may be taken as a single dose or may be divided into 2 to 4 daily administrations. A 3-divided manner is preferred. The unit dose of ibuprofen is preferably 60 to 200 mg, more preferably 150 mg or 200 mg.

The ibuprofen amount of the pharmaceutical composition of the present invention is not particularly limited, and the lower limit of the ibuprofen amount (with respect to the total mass of the composition) is preferably 10 mass%, more preferably 15 mass%, still more preferably 20 mass%, yet more preferably 40 mass%, yet more preferably 45 mass%, particularly preferably 50 mass%. The upper limit is preferably 95 mass%, more preferably 90 mass%, still more preferably 85 mass%.

Scopolamine butylbromide, employed in the pharmaceutical composition of the present invention, is a known compound, and may be produced through a known method or may be commercially available. In the present invention, scopolamine butylbromide listed in an official document or the like published in countries, regions, etc. is preferred. Examples of the official document include the Japanese Pharmacopoeia, the United States Pharmacopoeia, the British Pharmacopoeia, the European Pharmacopoeia, and the Pharmacopoeia of the People's Republic of China. Scopolamine butylbromide listed in the Japanese Pharmacopoeia assumes white crystals or crystalline powder.

The scopolamine butylbromide amount of the pharmaceutical composition of the present invention may be determined in accordance with the sex, age, symptoms, etc. of a patient in need of the composition and in appropriate consideration of the daily dose. The scopolamine butylbromide amount is preferably adjusted so as to attain a scopolamine butylbromide daily dose of 3 to 1,000 mg, more preferably 5 to 500 mg, still more preferably 30 to 100 mg. The daily dose may be taken as a single dose or may be divided into 2 to 5 times. A 3-divided manner is preferred. The unit dose of scopolamine butylbromide is preferably 10 to 20 mg, more preferably 10 mg or 20 mg.

The ratio of ibuprofen amount to scopolamine butylbromide amount of the pharmaceutical composition of the present invention may be determined in appropriate consideration of the aforementioned daily dose of each ingredient. The amount of scopolamine butylbromide with respect to 1 part by mass of ibuprofen is preferably 0.0125 to 1 part by mass, more preferably 0.022 to 1 part by mass.

The scopolamine butylbromide amount of the pharmaceutical composition of the present invention is not particularly limited, and the lower limit of the scopolamine butylbromide amount (with respect to the total mass of the composition) is preferably 0.1 mass%, more preferably 0.5 mass%, still more preferably 1 mass%, yet more preferably 3 mass%, yet more preferably 3.2 mass%, particularly preferably 3.3 mass%. The upper limit is preferably 10 mass%, more preferably 8 mass%, still more preferably 6 mass%.

The "one or more species selected from the group consisting of caffeine, tranexamic acid or a salt thereof, acetaminophene, bromovaleryl urea and allylisopropylacetyl urea" employed in the pharmaceutical composition of the present invention is described hereinafter.

The aforementioned caffeine may be in the form of a complex salt (e.g., caffeine sodium benzoate (a complex salt of sodium benzoate and caffeine), or the like.

Specific examples of preferred caffeines include caffeine hydrate, anhydrous caffeine, caffeine sodium benzoate, and caffeine citrate. Of these, caffeine hydrate, anhydrous caffeine, and caffeine sodium benzoate are more preferred.

The caffeine amount of the pharmaceutical composition of the present invention may be determined in appropriate consideration of the ibuprofen-scopolamine butylbromide interaction inhibitory effect, and of the daily dose in accordance with the sex, age, symptoms, etc. of a patient in need of the composition. However, the caffeine amount is preferably adjusted so as to attain caffeine daily dose of 10 to 1,000 mg, more preferably 20 to 800 mg, still more preferably 40 to 600 mg.

The caffeine amount may be determined in appropriate consideration of the aforementioned dose. The lower limit of the caffeine amount of the pharmaceutical composition of the present invention (with respect to the total mass of the composition) is preferably 1 mass%, more preferably 5 mass%, still more preferably 10 mass%, yet more preferably 20 mass%. The upper limit is preferably 90 mass%, more preferably 85 mass%, still more preferably 80 mass%.

The ratio of ibuprofen amount to caffeine amount of the pharmaceutical composition of the present invention may be determined in appropriate consideration of the aforementioned daily dose of each ingredient. The amount of caffeine with respect to 1 part by mass of ibuprofen is 0.05 to 7 parts by mass, preferably 0.1 to 5.5 parts by mass, still more preferably 0.2 to 4 parts by mass.

The tranexamic acid or a salt thereof employed in the pharmaceutical composition of the present invention encompasses tranexamic acid, a pharmaceutically acceptable salt of tranexamic acid, and a solvate of tranexamic acid or a pharmaceutically acceptable salt of tranexamic acid with a solvent such as water or alcohol. These compounds are known compounds, and may be produced through a known method or may be commercially available. In the present invention, preferred are tranexamic acid and a salt thereof listed in an official document or the like published in countries, regions, etc. Examples of the official document include the Japanese Pharmacopoeia, the United States Pharmacopoeia, the British Pharmacopoeia, the European Pharmacopoeia, and the Pharmacopoeia of the People's Republic of China. When the pharmaceutical composition of the present invention is used as an antipyretic analgesic, an all-in-one cold and flu drug, or a like drug, the tranexamic acid or a salt thereof is preferably tranexamic acid.

The tranexamic acid (or a salt thereof) amount of the pharmaceutical composition of the present invention may be determined in appropriate consideration of the ibuprofen-scopolamine butylbromide interaction inhibitory effect, and of the daily dose in accordance with the sex, age, symptoms, etc. of a patient in need of the composition. However, the tranexamic acid (or a salt thereof) amount is preferably adjusted so as to attain a free tranexamic acid daily dose of 50 to 2,000 mg, more preferably 70 to 750 mg, still more preferably 400 to 750 mg.

The tranexamic acid (or a salt thereof) amount may be determined in appropriate consideration of the aforementioned dose. The tranexamic acid (or a salt thereof) amount of the pharmaceutical composition of the present invention (with respect to the total mass of the composition) is preferably 20 to 95 mass%, more preferably 30 to 85 mass%, still more preferably 40 to 80 mass%.

The ratio of ibuprofen amount to tranexamic acid (or a salt thereof) amount of the pharmaceutical composition of the present invention may be determined in appropriate consideration of the aforementioned daily dose of each ingredient. The amount of tranexamic acid or a salt thereof, as reduced to the amount of free tranexamic acid, with respect to 1 part by mass of ibuprofen, is 0.3 to 15 parts by mass, preferably 0.4 to 5 parts by mass, still more preferably 0.8 to 5 parts by mass.

The isovaleryl urea derivative employed in the pharmaceutical composition of the present invention is one species selected from among the group consisting of bromovaleryl urea and allylisopropylacetyl urea. Since an isovaleryl urea derivative, having an asymmetric carbon atom, includes several optical isomers. The present invention encompasses any isomer thereof, and the isovaleryl urea derivative may be a single optical isomer or a mixture of a plurality of isomers. These isomers are known compounds, and may be produced through a known method or may be commercially available. The isovaleryl urea derivative is preferably an isovaleryl urea derivative listed in an official document or the like published in countries, regions, etc. Examples of the official document include the Japanese Pharmacopoeia, the United States Pharmacopoeia, the British Pharmacopoeia, the European Pharmacopoeia, and the Pharmacopoeia of the People's Republic of China.

When the pharmaceutical composition of the present invention is used as an antipyretic analgesic, an all-in-one cold and flu drug, or a like drug, the isovaleryl urea derivative employed in the pharmaceutical composition of the present invention is preferably allylisopropylacetyl urea.

The isovaleryl urea derivative amount of the pharmaceutical composition of the present invention is not particularly limited, and the amount may be determined in appropriate consideration of the ibuprofen-scopolamine butylbromide interaction inhibitory effect, and of the daily dose in accordance with the sex, age, symptoms, etc. of a patient in need of the composition. In the case where the isovaleryl urea derivative is bromovaleryl urea, the bromovaleryl urea derivative amount is preferably adjusted so as to attain a free bromovaleryl urea daily dose of 10 to 1,000 mg, more preferably 30 to 800 mg, still more preferably 60 to 600 mg. In the case where the isovaleryl urea derivative is allylisopropylacetyl urea, the allylisopropylacetyl urea derivative amount is preferably adjusted so as to attain a free allylisopropylacetyl urea daily dose of 1 to 500 mg, more preferably 10 to 300 mg, still more preferably 20 to 180 mg.

The isovaleryl urea derivative amount may be determined in appropriate consideration of the aforementioned dose. The lower limit of the isovaleryl urea derivative amount of the pharmaceutical composition of the present invention (with respect to the total mass of the composition) is preferably 5 mass%, more preferably 10 mass%, still more preferably 15 mass%, yet more preferably 20 mass%. The upper limit is preferably 90 mass%, more preferably 85 mass%, still more preferably 80 mass%.

The ratio of ibuprofen amount to isovaleryl urea derivative amount of the pharmaceutical composition of the present invention may be determined in appropriate consideration of the aforementioned daily dose of each ingredient. In the case where the isovaleryl urea derivative is bromovaleryl urea, the amount of bromovaleryl urea with respect to 1 part by mass of ibuprofen, is 0.05 to 7 parts by mass, preferably 0.2 to 5.5 parts by mass, still more preferably 0.4 to 4 parts by mass. In the case where the isovaleryl urea derivative is allylisopropylacetyl urea, the amount of allylisopropylacetyl urea with respect to 1 part by mass of ibuprofen, is 0.005 to 3.5 parts by mass, preferably 0.05 to 2.0 parts by mass, still more preferably 0.1 to 1.5 parts by mass.

The acetaminophene employed in the pharmaceutical composition of the present invention is a known compound, and may be produced through a known method or may be commercially available. The acetaminophene is preferably an acetaminophene product listed in an official document or the like published in countries, regions, etc. Examples of the official document include the Japanese Pharmacopoeia, the United States Pharmacopoeia, the British Pharmacopoeia, the European Pharmacopoeia, and the Pharmacopoeia of the People's Republic of China.

The acetaminophene amount of the pharmaceutical composition of the present invention is not particularly limited, and the amount may be determined in appropriate consideration of the ibuprofen-scopolamine butylbromide interaction inhibitory effect, and of the daily dose in accordance with the sex, age, symptoms, etc. of a patient in need of the composition. The acetaminophene amount is preferably adjusted so as to attain an acetaminophene daily dose of 10 to 1,500 mg, more preferably 50 to 1,200 mg, still more preferably 100 to 1,000 mg.

The acetaminophene amount may be determined in appropriate consideration of the aforementioned dose. The lower limit of the acetaminophene amount of the pharmaceutical composition of the present invention (with respect to the total mass of the composition) is preferably 5 mass%, more preferably 20 mass%, still more preferably 30 mass%, yet more preferably 35 mass%. The upper limit is preferably 90 mass%, more preferably 88 mass%, still more preferably 86 mass%.

The ratio of ibuprofen amount to acetaminophene amount of the pharmaceutical composition of the present invention may be determined in appropriate consideration of the aforementioned daily dose of each ingredient. The amount of acetaminophene, with respect to 1 part by mass of ibuprofen, is 0.05 to 10 parts by mass, preferably 0.3 to 8 parts by mass, still more preferably 0.6 to 7 parts by mass.

The pharmaceutical composition of the present invention may be produced through a known method; e.g., a method described in "The Japanese Pharmacopoeia, 16th Edition, General Rules for Preparations" by use of known additives for pharmaceutical preparation, whereby preparations of various dosage forms are produced.

The dosage form of the pharmaceutical composition of the present invention is not particularly limited. Preferred is a solid preparation containing ibuprofen, scopolamine butylbromide, and one or more species selected from the group consisting of caffeine, tranexamic acid or a salt thereof, acetaminophene, and an isovaleryl urea derivative which is bromovaleryl urea or allylisopropylacetyl urea (hereinafter the third ingredient(s) may be referred to as "caffeine, etc."), from the viewpoints of ease of taking the composition, dose control, etc. Specific examples of the solid preparation include peroral preparations including tablets (including peroral tablets such as orally disintegrating tablets, chewable tablets, dispersion tablets, soluble tablets, troches, sublingual tablets, buccal tablets, adhesive tablets, and gummy agents), capsules, pills, granules, fine granules, powders, dry syrups, and peroral jellies; and parenteral preparations such as suppositories, vaginal tablet, and vaginal suppository. Among them, peroral solid preparations are preferred. The pharmaceutical composition of the present invention may be coated through a known method with sugar coating, film coating, or a similar coating material.

In the present invention, known additives may be appropriately used for producing pharmaceutical preparations of various dosage forms. Among such additives, a water-absorbent polymer is preferably used. Water-absorbent polymer can reduce interaction between ibuprofen and scopolamine butylbromide. The type of water-absorbent polymer is not particularly limited, so long as the polymer can reduce the interaction. Examples of the water-absorbent polymer include a hygroscopic cellulose derivative or a salt thereof, a hygroscopic 1-vinyl-2-pyrrolidone polymer, and a hygroscopic starch derivative or a salt thereof. Of these, a hygroscopic starch derivative or a salt thereof is preferred.

Examples of the hygroscopic cellulose derivative or a salt thereof include a cellulose derivative in which OH groups have been etherified, an ester of the etherified cellulose, a cross-linked polymer of any of these, and a salt of any of these. Examples of the hygroscopic cellulose derivative or a salt thereof include carmellose, carmellose potassium, carmellose calcium, carmellose sodium, croscarmellose sodium, hydroxyethylcellulose, and hydroxypropylmethylcellulose acetate succinate. Of these, carmellose, carmellose potassium, carmellose calcium, carmellose sodium, and croscarmellose sodium are particularly preferred. These compounds are known compounds, and may be produced through a known method or may be commercially available.

Examples of the hygroscopic 1-vinyl-2-pyrrolidone polymer include 1-vinyl-2-pyrrolidone liner-chain polymer and 1-vinyl-2-pyrrolidone cross-linked polymer. Examples of the 1-vinyl-2-pyrrolidone polymer include polyvinylpyrrolidone K17, polyvinylpyrrolidone K25, polyvinylpyrrolidone K30, polyvinylpyrrolidone K90, and crospovidone. Examples of the hygroscopic starch derivative or a salt thereof include starch carboxymethyl ether and a salt thereof, such as carboxymethyl starch sodium.

The water-absorbent polymer amount of the pharmaceutical composition of the present invention may be determined in appropriate consideration of, for example, the type of water-absorbent polymer used for reducing the interaction between ibuprofen and scopolamine butylbromide. In one embodiment, the amount of water-absorbent polymer, with respect to 1 part by mass of ibuprofen, is preferably adjusted to 0.01 to 3 parts by mass, more preferably 0.02 to 2 parts by mass.

The present inventors have first found that a certain interaction results between ibuprofen and scopolamine butylbromide which are contained in a pharmaceutical composition, when ibuprofen is brought in contact with scopolamine butylbromide. Based on this finding, the inventors have successfully reduced the interaction between the two ingredients by substantially avoiding contact between ibuprofen and scopolamine butylbromide. In other words, one embodiment of the present invention is a pharmaceutical composition containing ibuprofen and scopolamine butylbromide in such a manner that the two ingredients are substantially in non-contact with each other, and further containing caffeine, etc.

As used herein, the expression "two ingredients are substantially in non-contact with each other" means that ibuprofen and scopolamine butylbromide are incorporated into the pharmaceutical composition in such a non-contact state that no interaction occurs between the two ingredients. Preferably, ibuprofen and scopolamine butylbromide are incorporated into the composition in a completely non-contact state.

One embodiment of the solid preparation according to the present invention contains (A) ibuprofen or a solid composition containing ibuprofen, and (B) scopolamine butylbromide or a solid composition containing scopolamine butylbromide, in which ibuprofen and scopolamine butylbromide are isolated from each other by means of an ingredient or ingredients forming the solid composition. The solid composition or compositions is or are in the form of powder, granules, tablet, etc.

Specific embodiments of the solid preparation include the following (I) to (VIII). As described above, these solid preparations may be produced through a known method; e.g., a method described in "The Japanese Pharmacopoeia, 16th Edition, General Rules for Preparations" by use of any known additives for pharmaceutical preparation.
(I) A preparation produced by granulating any one of ibuprofen and scopolamine butylbromide through an appropriate technique, adding the granulate to the non-granulated counterpart, to thereby provide a powder or a granule, and optionally coating the granulated product with an appropriate material through an appropriate technique. When the caffeine, etc. of the present invention is used, the caffeine, etc. may optionally be incorporated into the granulated product.
(II) A preparation produced by granulating ibuprofen and scopolamine butylbromide individually through an appropriate technique, and optionally coating a powder or a granule containing the granulated product with an appropriate material through an appropriate technique. The caffeine, etc. may optionally be incorporated into one granulated product or both granulated products, or incorporated into the solid preparation such that the ingredient is separated with the granulated product(s).
(III) A capsule agent including the powder, granule, etc. produced in the aforementioned (I) or (II).
(IV) A tablet formed by pelletizing, through a similar appropriate technique, the granulated product, etc. obtained in the aforementioned (I) or (II). Pelletizing may be performed by means of compression or another appropriate technique for shaping into a certain form.
(V) A multilayer tablet produced so that contact between ibuprofen and scopolamine butylbromide is substantially avoided, and a coated multilayer tablet produced through an appropriate technique. In a preferred embodiment of the multilayer tablet, ibuprofen and scopolamine butylbromide are included in different layers. A more preferred embodiment of the multilayer tablet is a multilayer tablet having 3 or more layers in which an ibuprofen-containing layer and a scopolamine butylbromide-containing layer are not in contact with each other. As an ibuprofen source and a scopolamine butylbromide source, the granulated products produced in (I) and (II) above may be used. In the multilayer tablet, the caffeine, etc. may be incorporated into an ibuprofen-containing layer or a scopolamine butylbromide-containing layer or may be incorporated into both layers. Alternatively, the caffeine, etc. may be incorporated into an intermediate layer other than these layers.
(VI) A dry coated tablet having a nucleus tablet (also called core tablet or center tablet) containing any one of ibuprofen and scopolamine butylbromide so that contact between ibuprofen and scopolamine butylbromide is substantially avoided, and a further coated dry coated tablet produced through an appropriate technique. As an ibuprofen source and a scopolamine butylbromide source, the granulated products produced in (I) and (II) above may be used. In the dry coated tablet, the caffeine, etc. may be incorporated into the nucleus tablet or into an outer layer, or divided and incorporated into both the nucleus tablet and an outer layer.
(VII) An inclusion compound preparation containing, instead of the granulated product produced in (I) or (II), ibuprofen and/or scopolamine butylbromide clathrated with a cyclodextrin such as α-cyclodextrin, β-cyclodextrin, or γ-cyclodextrin, or with a similar material. The caffeine, etc. may be present in the vicinity of one inclusion compound or of both inclusion compounds.
(VIII) A preparation having a core preparation produced through a conventional technique and containing any one of ibuprofen and scopolamine butylbromide, and a sugar coating layer or a film coating layer containing the counterpart, so that contact between ibuprofen and scopolamine butylbromide is substantially avoided. In the case where the dosage form is a tablet, the preparation is also called a sugar-coated tablet or a film-coated tablet. The caffeine, etc. may be incorporated into the core preparation produced through a conventional technique or into the sugar coating layer or the film coating layer. The caffeine, etc. may be divided and incorporated into both the sugar coating layer and the film coating layer. In the preparation, the caffeine, etc. may be contained in both the sugar coating layer and the film coating layer.

The granulated products of (I) and (II) above may be obtained through a known granulation technique such as extrusion granulation, oscillation granulation, stirring granulation, fluidized bed granulation, spray dry granulation, crushing granulation, or melt granulation, by use of known additives for pharmaceutical preparation in accordance with needs. In the present invention, the granulated product containing ibuprofen and that containing scopolamine butylbromide may be produced through the same granulation technique or through granulation techniques that are different from each other.

The granulated product containing ibuprofen may be produced through granulation of a mixture containing ibuprofen, a fluidizing agent (e.g., light anhydrous silicic acid or hydrous silica), a binder (e.g., hydroxypropylcellulose or hypromellose), etc. Alternatively, the granulated product may also be produced through granulation of ibuprofen by use of a binder solution in which a fluidizing agent or the like are dispersed. A known granulation technique may be employed. A commercial granulated product containing ibuprofen may also be used. Examples of such commercial products include ibuprofen granules 20% "Tatsumi" (product of Tastumi Kagaku Co., Ltd.), BRUFEN (registered trademark) granules 20% (product of Kaken Pharmaceutical Co., Ltd.), and Landelun (registered trademark) granules 20% (product of Tsuruhara Pharmaceutical Co., Ltd.). When scopolamine butylbromide is used instead of ibuprofen, a granulated product containing scopolamine butylbromide may be produced through the same technique, and a commercial granulated product may also be used.

The pharmaceutical composition of the present invention may further contain, other than ibuprofen and scopolamine butylbromide, one or more species selected from the group consisting of an antipyretic analgesic, an antihistamic agent, an antitussive, noscapine, a bronchodilator, an expectorant, a vitamin, an anti-inflammatory agent, a gastric mucosal protector, an anticholinergic agent, a crude drug, a *Kampo* formulation, etc.

Examples of the antipyretic analgesic include aspirin, aspirin aluminum, isopropylantipyrine, ethenzamide, sasapyrine, salicylamide, sodium salicylate, tiaramide hydrochloride, lactylphenetidine, and loxoprofen sodium hydrate.

Examples of the antihistamic agent include azelastine hydrochloride, alimemazine tartrate, isothipendyl hydrochloride, iproheptine hydrochloride, epinastine hydrochloride, emedastine fumarate, carbinoxamine diphenyldisulfonate, carbinoxamine maleate, clamastine fumarate, dl-chlorphenyramine maleate, d-chlorphenyramine maleate, ketotifen fumarate, difeterol hydrochloride, difeterol phosphate, diphenylpyraline hydrochloride, diphenylpyraline theoclate, diphenhydramine hydrochloride, diphenhydramine salicylate, diphenhydramine tannate, triprolidine hydrochloride, tirpelennamine hydrochloride, thonzylamine hydrochloride, fenethazine hydrochloride, promethazine hydrochloride, promethazine methylenedisalicylate, mequitazine, methdilazine hydrochloride, mebhydrolin napadisilate, and emedastine fumarate.

Examples of the antitussive include alloclamide hydrochloride, eprazinone hydrochloride, carbetapentane citrate, cloperastine hydrochloride, cloperastine fendizoate, codeine phosphate, dihydrocodeine phosphate, sodium dibunate, dimemorfan phosphate, dextromethorphan hydrobromide, dextromethorphan phenolphthalinate, tipepidine citrate, and tipepidine hibenzate.

Examples of the noscapine species include noscapine hydrochloride and noscapine.

Examples of the bronchodilator include trimetoquinol hydrochloride, phenylpropanolamine hydrochloride, phenylephrine hydrochloride, pseudoephedrine hydrochloride, pseudoephedrine sulfate, methylephedrine, dl-methylephedrine hydrochloride, 1-methylephedrine hydrochloride, dl-methylephedrine saccharic acid salt, and methoxyphenamine hydrochloride.

Examples of the expectorant include ambroxol hydrochloride, foeniculated ammonia spirit, ethylcysteine hydrochloride, ammonium chloride, carbocysteine, guaifenesine, potassium guaiacolsulfonate, potassium cresolsulfonate, bromhexine hydrochloride, methylcysteine hydrochloride, 1-menthol, and lysozyme hydrochloride.

Examples of the vitamin include vitamin B₁, vitamin B₂, vitamin B₅, vitamin B₆, vitamin B₁₂, vitamin C, hesperidin, derivatives thereof, and salts of any of these (e.g., thiamine, thiamine chloride hydrochloride, thiamine nitrate, dicethiamine hydrochloride, cetotiamine hydrochloride, fursultiamine, fursultiamin hydrochloride, octothiamine, cycotiamine, thiamine disulfide, bisibuthiamine, bisbentiamine, prosultiamine, benfotiamine, riboflavin, riboflavin phosphate, riboflavin butyrate, riboflavin sodium phosphate, panthenol, pantethine, calcium pantothenate, sodium pantothenate, pyridoxine hydrochloride, pyridoxal phosphate, cyanocobalamin, mecobalamin, ascorbic acid, sodium ascorbate, calcium ascorbate, and hesperidin).

Examples of the anti-inflammatory agent include glycyrrhizic acid, derivatives thereof, and salts of any of these (e.g., dipotassium glycyrrhizate and monoammonium glycyrrhizate), Seaprose, semi-alkaline proteinase, serrapaptase, proctase, pronase, and bromelain.

Examples of the gastric mucosal protector include aminoacetic acid, Aldioxa, magnesium silicate, Gefarnate, synthetic aluminum silicate, synthetic hydrotalcite, magnesium oxide, dihydroxyaluminum aminoacetate (aluminum glycinate), aluminum hydroxide gel, aluminum hydroxide-magnesium carbonate mixture dry gel, aluminum hydroxide-sodium hydrogencarbonate coprecipitation product, aluminum hydroxide-sodium carbonate-magnesium carbonate coprecipitation product, magnesium hydroxide-potassium aluminum sulfate coprecipitation product, scralfate, cetraxate hydrochloride, sofalcone, magnesium carbonate, teprenone, potassium copper chlorophyllin, sodium copper chlorophyllin, magnesium metasilicate aluminate, and methylmethionine sulfonium chloride.

Examples of the anticholinergic agent include oxyphencyclimine hydrochloride, dicyclomine hydrochloride, methixene hydrochloride, scopolamine hydrobromide, datura extract, tipepidium bromide, methylatropine bromide, methylanisotropine bromide, methylscopolamine bromide, methyl-1-hyoscyamine bromide, methylbenactyzium bromide, pirenzepine hydrochloride, belladonna alkaloid, belladonna extract, belladonna total alkaloid, isopropamide iodide, diphenylpiperidinonemethyldioxolane iodide, scopolia extract, scopolia rhizome, and scopolia rhizome total alkaloid citrate.

Examples of the crude drug include crude drugs such as Mallotus bark, Gambir, Epimedium herb, Fennel, Turmeric, Corydalis tuber, Plectranthus, Scutellaria root, Polygonatum rhizome, Phellodendron bark, Cherry bark, Coptis rhizome, Polygala root, Zedoary, Japanese Valerian, German chamomile, Tricosanthes, GLycyrrhiza, Platycodon root, Apricot kernel, Lycium fruit, Lycium leaf, Schizonepeta spike, Cinnamon bark, Cassia seed, Gentian, Geranium herb, Cyperus rhizome, Oriental Bezoar, Schisandra fruit, Asiasarum root, Zanthoxylum fruit, Tartarian Aster, Lycium bark, Peony root, Musk, Adenophora polymorpha root, Plantago seed, Plantago herb, beast's gall (including bear's gall), Ginger, Oligochaeta, Magnolia flower, Lycoris radiata, Senega, Cnidium rhizome, Peucedanum root, Swertia herb, Atractylodes Lancea rhizome, Mulberry bark, Perilla herb, Allium sativum, Panax Japonicus rhizome, Clove, Citrus Unshiu peel, Japanese Angelica root, Ipecac, Nandia Domestica fruit, Ginseng, Fritillaria bulb, Ophiopogon tuber, Mentha herb, Pinellia tuber, Saffron, Gloydius blomhoffii, Angelica Dahurica root, Atractylodes rhizome, Poria Sclerotium, Mautan bark, Oyster shell, Ephedra herb, and young deer horn; and extracts (extract, tincture, dry extract, etc.) thereof.

Examples of the *Kampo* formulation include *Kakkonto, Keisito, Kososan, Saikokeisito, Shosaikoto, Shoseiryuto, Bakumondoto, Hangekobokuto,* and *Maoto.*

In the present invention, the pharmaceutical composition of the present invention is used as an antipyretic analgesic, an all-in-one cold and flu drug, or a like drug. Thus, specific examples of pharmaceutical ingredients other than ibuprofen and scopolamine butylbromide include antipyretic analgesics such as isopropylantipyrine and ethenzamide; antihistamic agents; and gastric mucosal protectors such as aluminum hydroxide dry gel, synthetic hydrotalcite, and magnesium oxide.

Since the pharmaceutical composition of the present invention is used as an antipyretic analgesic, an all-in-one cold and flu drug, or a like drug, examples of the effects (indications) of the composition include mitigation of headache, toothache, pain after tooth extraction, throat pain, earache, joint pain, neuralgia, backache, muscle pain, shoulder stiffness pain, contusion pain, fracture pain, sprain pain, menstrual period pains), traumatic pain, chill and fever, and various cold symptoms (sore throat, chill, fever, headache, joint pain, and muscle pain).

### Examples

The present invention will next be described in detail by way of examples. Unless otherwise specified, the unit "part(s) by mass" will be represented by "part(s)."

### Test Example 1

### Interaction study (1)

A mixture of ibuprofen (150 parts) and scopolamine butylbromide (10 parts) was added to a glass bottle and stored at 50°C (Reference 1). As comparative references, ibuprofen (Control 1) or scopolamine butylbromide (Control 2) was added to a glass bottle of the same type and stored at 50°C. The condition of the contents of each glass bottle was observed immediately after storage, after storage for 2 weeks, and after storage for 4 weeks. Table 1 shows the results.

**[Table 1]**

| | Start of storage | 2 weeks | 4 weeks |
|---|---|---|---|
| Control 1 | white powder | white powder | white powder |
| Control 2 | white powder | white powder | white powder |
| Reference 1 | white powder | wetted white powder | wetted pale yellow powder |

As is clear from Table 1, during storage of the mixture of ibuprofen and scopolamine butylbromide, the mixture was wetted and discolored (Reference 1). However, during storage of ibuprofen as a single component and of scopolamine butylbromide as a single component, each component exhibited no change in appearance (Controls 1 and 2).

Thus, the change in state of the mixture was found to result from an interaction between ibuprofen and scopolamine butylbromide.

### Test Example 2

### Interaction study (2)

A mixture of ibuprofen (150 parts) and scopolamine butylbromide (10 parts) was added to a glass bottle and stored at 40°C (Reference 2). As comparative references, ibuprofen (Control 3) or scopolamine butylbromide (Control 4) was added to a glass bottle of the same type and stored at 40°C.

The condition of the contents of each glass bottle was observed immediately after storage, and after storage for 6 months. Table 2 shows the results.

**[Table 2]**

| | Start of storage | 6 months |
|---|---|---|
| Control 3 | white powder | white powder |
| Control 4 | white powder | white powder |
| Reference 2 | white powder | wetted pale yellow powder |

As is clear from Table 2, after storage of the mixture of ibuprofen and scopolamine butylbromide at 40°C for 6 months, the mixture was wetted and discolored (Reference 2). In contrast, during storage of ibuprofen as a single component and of scopolamine butylbromide as a single component, each component remained the state immediately after the start of storage and exhibited no change in appearance (Controls 3 and 4).

Thus, the change in state of the mixture was found to result from an interaction between ibuprofen and scopolamine butylbromide.

### Test Example 3

### Interaction study (3)

A mixture of ibuprofen (450 parts) and scopolamine butylbromide (30 parts) was added to a glass bottle and stored at 60°C (Reference 3) .

Separately, A mixture of ibuprofen (450 parts), scopolamine butylbromide (30 parts), and tranexamic acid (420 parts) was added to a glass bottle and stored at 60°C (Example 1).

The procedure of Example 1 was repeated except that tranexamic acid was substituted with anhydrous caffeine (150 parts), and the mixture was added to a glass bottle and stored at 60°C (Example 2).

The procedure of Example 1 was repeated except that tranexamic acid was substituted with acetaminophene (400 parts), and the mixture was added to a glass bottle and stored at 60°C (Example 3).

The procedure of Example 1 was repeated except that tranexamic acid was substituted with allylisopropylacetyl urea (180 parts), and the mixture was added to a glass bottle and stored at 60°C (Example 4).

The state of the contents of each glass bottle was evaluated immediately after storage, and after one week storage. Table 3 shows the results.

**[Table 3]**

| | Start of storage | 1 week |
|---|---|---|
| Reference 3 | white powder | solidified white powder |
| Example 1 | white powder | white powder |
| Example 2 | white powder | white powder |
| Example 3 | white powder | white powder |
| Example 4 | white powder | white powder |

As is clear from Table 3, the state of the mixture of ibuprofen and scopolamine butylbromide after storage at 60°C for one week was a solidified mixture (Reference 3).

In contrast, the mixture of ibuprofen and scopolamine butylbromide with tranexamic acid (Example 1), with anhydrous caffeine (Example 2), with acetaminophene (Example 3), or allylisopropylacetyl urea (Example 4), remained the state immediately after the start of storage even after the storage for one week, and exhibited no change.

Therefore, the interaction between ibuprofen and scopolamine butylbromide was found to be suppressed by tranexamic acid or a salt thereof, caffeine, acetaminophene, or an isovaleryl urea derivative.

### Production Example 1

Ibuprofen (150 parts), hydroxypropylmethylcellulose (12 parts), croscarmellose sodium (36 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), tranexamic acid (250 parts), hydroxypropylcellulose (10 parts), and lactose hydrate (36 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each attaining an ibuprofen daily dose of 450 mg, a scopolamine butylbromide daily dose of 30 mg, and a tranexamic acid daily dose of 750 mg.

### Production Example 2

Ibuprofen (150 parts), hydroxypropylmethylcellulose (12 parts), croscarmellose sodium (27 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), anhydrous caffeine (50 parts), hydroxypropylcellulose (5 parts), and lactose hydrate (30 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, a scopolamine butylbromide amount of 10 mg, and an anhydrous caffeine amount of 50 mg.

### Production Example 3

Ibuprofen (150 parts), hydroxypropylmethylcellulose (12 parts), carmellose (27 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), allylisopropylacetyl urea (60 parts), hydroxypropylcellulose (4 parts), and lactose hydrate (31 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, a scopolamine butylbromide amount of 10 mg, and an allylisopropylacetyl urea amount of 60 mg.

### Production Example 4

Ibuprofen (150 parts), hydroxypropylmethylcellulose (12 parts), carmellose (32 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), acetaminophene (134 parts), hydroxypropylcellulose (8 parts), and lactose hydrate (38 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, a scopolamine butylbromide amount of 10 mg, and an acetaminophene amount of 134 mg.

### Production Example 5 (reference example)

Ibuprofen (150 parts), hydroxypropylmethylcellulose (12 parts), croscarmellose sodium (22 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), magnesium oxide (33 parts), hydroxypropylcellulose (4 parts), and lactose hydrate (33 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, and a scopolamine butylbromide amount of 10 mg.

### Production Example (reference examples)

Ibuprofen (150 parts), hydroxypropylmethylcellulose (12 parts), croscarmellose sodium (44 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), synthetic hydrotalcite (178.7 parts), hydroxypropylcellulose (12 parts), and lactose hydrate (33 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, and a scopolamine butylbromide amount of 10 mg.

### Production Example 7

Ibuprofen (150 parts), hydroxypropylmethylcellulose (12 parts), carmellose (44 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), tranexamic acid (250 parts), hydroxypropylcellulose (12 parts), and lactose hydrate (33 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each attaining an ibuprofen daily dose of 450 mg, a scopolamine butylbromide daily dose of 30 mg, and a tranexamic acid daily dose of 750 mg.

### Production Example 8

Ibuprofen (150 parts), hydroxypropylmethylcellulose (12 parts), carmellose (44 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), tranexamic acid (250 parts), magnesium oxide (33 parts), hydroxypropylcellulose (12 parts), and lactose hydrate (33 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each attaining an ibuprofen daily dose of 450 mg, a scopolamine butylbromide daily dose of 30 mg, and a tranexamic acid daily dose of 750 mg.

### Production Example 9

Ibuprofen (150 parts), hydroxypropylmethylcellulose (12 parts), croscarmellose sodium (27 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), anhydrous caffeine (50 parts), magnesium oxide (33 parts), hydroxypropylcellulose (5 parts), and lactose hydrate (37 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, a scopolamine butylbromide amount of 10 mg, and an anhydrous caffeine amount of 50 mg.

### Production Example 10

Ibuprofen (150 parts), hydroxypropylmethylcellulose (12 parts), croscarmellose sodium (27 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), allylisopropylacetyl urea (60 parts), magnesium oxide (33 parts), hydroxypropylcellulose (5 parts), and lactose hydrate (37 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, a scopolamine butylbromide amount of 10 mg, and an allylisopropylacetyl urea amount of 60 mg.

### Production Example 11 (reference example)

Ibuprofen (150 parts), hydroxypropylmethylcellulose (12 parts), carmellose (22 parts), and light anhydrous silicic acid (6 parts) were mixed together, and the mixture was granulated through kneading with purified water, and the grain size was adjusted, to thereby yield a first granulation product. Separately, scopolamine butylbromide (10 parts), synthetic hydrotalcite (33 parts), hydroxypropylcellulose (5 parts), and lactose hydrate (32 parts) were mixed together, and the mixture was granulated through kneading with ethanol, and the grain size was adjusted, to thereby yield a second granulation product. To the thus-obtained two granulation products, talc (10 parts) was added, to thereby form granules to be pelletized.

The granules were pelletized, to thereby produce tablets each containing an ibuprofen amount of 150 mg, and a scopolamine butylbromide amount of 10 mg.

### Production Example 12

Hydroxypropylmethylcellulose (10 parts) and triethyl citrate (1 part) are dissolved in purified water (115 parts), and titanium oxide (2 parts) was dispersed in the resultant solution, to thereby prepare a film coating liquid. The film coating liquid was sprayed, by means of a coating apparatus, onto the core tablets produced in Production Example 1 to 11 so that one single core tablet had a film layer of 10 mg, to thereby yield film-coated products of the tablets produced in Production Examples 1 to 11.

### Test Example 4

The film-coated products of the tablets produced in Production Examples 1 to 11, yielded in Production Example 12, were packed in press through packs (PTPs) (10 tablets/PTP sheet). Each PTP sheet was packed with an aluminum pillow pack and was stored at 40°C for 6 months.

After storage for six months, no change was observed in appearance of each film-coated tablet before and after the storage.

### Industrial Applicability

The present invention enables provision of a pharmaceutical composition which contains ibuprofen and scopolamine butylbromide and which has storage stability.

In addition, since the pharmaceutical composition of the present invention contains ibuprofen, scopolamine butylbromide, and caffeine, etc., which exhibit central nervous system exciting activity, cardiotonic/diuretic activity, gastric secretion promoting activity, smooth muscle relaxing activity, etc., the composition serves as an excellent antipyretic analgesic or all-in-one cold and flu drug.

## Claims

1. A pharmaceutical composition comprising (A) ibuprofen, (B) scopolamine butylbromide, and (C) one or more species selected from the group consisting of caffeine, tranexamic acid or a salt thereof, acetaminophene, bromovaleryl urea and allylisopropylacetyl urea,
wherein the amount of the one or more species (C) with respect to 1 part by mass of ibuprofen is as follows:
0.05 to 7 parts by mass of caffeine,
0.3 to 15 parts by mass of tranexamic acid or a salt thereof,
0.05 to 10 parts by mass of acetaminophene,
0.05 to 7 parts by mass bromovaleryl urea, and
0.005 to 3.5 parts by mass allylisopropylacetyl urea.

2. A pharmaceutical composition according to claim 1, wherein the caffeine is one or more species selected from the group consisting of caffeine hydrate, anhydrous caffeine, caffeine sodium benzoate, and caffeine citrate.

3. A pharmaceutical composition according to claim 1 or 2, wherein the tranexamic acid or a salt thereof is tranexamic acid.

4. A pharmaceutical composition according to any one of claims 1 to 3, wherein the amount of ibuprofen administered to a patient in need thereof ranges from 10 to 3,000 mg per day.

5. A pharmaceutical composition according to any one of claims 1 to 4, wherein scopolamine butylbromide administered to a patient in need thereof ranges from 3 to 1,000 mg per day.

6. A pharmaceutical composition according to any one of claims 1 to 5, wherein the dosage form thereof is a solid preparation.

## Patentansprüche

1. Pharmazeutische Zusammensetzung umfassend (A) Ibuprofen, (B) Scopolaminbutylbromid und (C) ein oder mehrere Elemente ausgewählt aus der Gruppe bestehend aus Koffein, Tranexamsäure oder einem Salz derselben, Acetaminophen, Bromvalerylharnstoff (Bromisoval) und Allylisopropylacetylharnstoff (Apronal),
wobei die Menge des einen oder der mehreren Elemente (C) bezüglich 1 Gewichtsteil Ibuprofen wie folgt ist:
0,05 bis 7 Gewichtsteile Koffein,
0,3 bis 15 Gewichtsteile Tranexamsäure oder einem Salz derselben,
0,05 bis 10 Gewichtsteile Acetaminophen,
0,05 bis 7 Gewichtsteile Bromvalerylharnstoff und
0,005 bis 3.5 Gewichtsteile Allylisopropylacetylharnstoff.

2. Eine pharmazeutische Zusammensetzung gemäß Anspruch 1, worin das Koffein eines oder mehrere ausgewählt aus der Gruppe bestehend aus Koffeinhydrat, wasserfreiem Koffein, Koffein-Natriumbenzoat und Koffeincitrat ist.

3. Eine pharmazeutische Zusammensetzung gemäß Anspruch 1 oder 2, worin die Tranexamsäure oder deren Salz Tranexamsäure ist.

4. Eine pharmazeutische Zusammensetzung gemäß einem der Ansprüche 1 bis 3, worin die Menge an Ibuprofen, die einem Patienten verabreicht wird, der dies benötigt, von 10 bis 3,000 mg pro Tag beträgt.

5. Eine pharmazeutische Zusammensetzung gemäß einem der Ansprüche 1 bis 4, worin Scopolaminbutylbromid, das einem Patienten verabreicht wird, der dies benötigt, 3 bis 1,000 mg pro Tag beträgt.

6. Eine pharmazeutische Zusammensetzung gemäß einem der Ansprüche 1 bis 5, deren Darreichungsform ein festes Präparat ist.

## Revendications

1. Composition pharmaceutique comprenant (A) de l'ibuprofène, (B) du butylbromure de scopolamine, et (C) une ou plusieurs espèce(s) choisie(s) dans l'ensemble constitué par la caféine, l'acide tranéxamique ou un sel de celui-ci, l'acétaminophène, la bromovalérylurée et l'allylisopropylacétylurée,
dans laquelle la quantité de la ou des espèce(s) (C) pour 1 partie en masse d'ibuprofène est la suivante :
0,05 à 7 parties en masse de caféine,
0,3 à 15 parties en masse d'acide tranéxamique ou d'un sel de celui-ci,
0,05 à 10 parties en masse d'acétaminophène,
0,05 à 7 parties en masse de bromovalérylurée, et
0,005 à 3,5 parties en masse d'allylisopropylacétylurée.

2. Composition pharmaceutique selon la revendication 1, dans laquelle la caféine est une ou plusieurs espèce(s) choisie(s) dans l'ensemble constitué par l'hydrate de caféine, la caféine anhydre, le sodium-benzoate de caféine, et le citrate de caféine.

3. Composition pharmaceutique selon la revendication 1 ou 2, dans laquelle l'acide tranéxamique ou un sel de celui-ci est l'acide tranéxamique.

4. Composition pharmaceutique selon l'une quelconque des revendications 1 à 3, dans laquelle la quantité d'ibuprofène administrée à un patient en ayant besoin est située dans la plage allant de 10 à 3000 mg par jour.

5. Composition pharmaceutique selon l'une quelconque des revendications 1 à 4, dans laquelle le butylbromure de scopolamine administré à un patient en ayant besoin est situé dans la plage allant de 3 à 1000 mg par jour.

6. Composition pharmaceutique selon l'une quelconque des revendications 1 à 5, dans laquelle la forme posologique de celle-ci est une préparation solide.
